# EUROPEAN PATENT APPLICATION

(11) **EP 1 428 553 A1**
(43) Date of publication of application: **16.06.2004**
(21) Application number: 02760941.1
(22) Date of filing: 29.07.2002
(51) Int. Cl.: A61N 1/36

(54) **ADAPTIVE ELECTROSTIMULATOR**

(30) Priority: 23.08.2001 RU 2001123470
(71) Applicant: Nadtochiy, Alekcandr Ivanovich, Taganrog, 347900 (RU)
(72) Inventor: NADTOCHIY, Alekcandr Ivanovich, Taganrog, 347900 (RU); GRINBERG, Yakov Zalmanovich, Tagonrog, 347909 (RU); FINAEV, Valeriy Ivanovich, Taganrog, 347900 (RU); ZENKIN, Maksim Vitalievich, Taganrog, 347900 (RU); UVAROV, Sergey Nikolaevich, Taganrog, 347900 (RU)
(74) Representative: Andrae, Steffen, Dr.
(86) International application number: PCT/RU2002/000358
(87) International publication number: WO 2003/018118

(57) **Abstract**

The present invention relates to medical equipment, in particular, to electronic devices of electrostimulation of a human organism.

The device is designed for therapeutic noninvasive influence on the skin surface of a human being by electric pulses with the purpose of exerting regulating influence on physiological systems of an organism in a wide spectrum of pathologies and thus achieving the analgesic effect.

It may be used for medical and rehabilitation purposes, in medical establishments, at home and any other conditions (during a business trip and when traveling, in a car, onboard a plane, etc.), with the purpose of relieving any type of pain, treatment of respiratory diseases (a cold, quinsy and other), in case of dyspeptic disorders (a diarrhea, a lock, a nausea, etc.), in urgent situations.

## Description

### Technical field

The invention concerns the area of medical equipment, in particular, to electronic devices of electrostimulation of a human organism.

The device is designed for therapeutic noninvasive influence on the skin surface of a human being by electric pulses with the purpose of exerting regulating influence on physiological systems of an organism in a wide spectrum of pathologies and thus achieving the analgesic effect.

It may be used for medical and rehabilitation purposes, in medical establishments, at home and any other conditions (during a business trip and when traveling, in a car, onboard a plane, etc.), with the purpose of relieving any type of pain, treatment of respiratory diseases (a cold, quinsy and other), in case of dyspeptic disorders (a diarrhea, a lock, a nausea, etc.), in urgent situations.

The device can be used by the medical personnel and doctors of various specialities.

### Prior art

There is a known electrostimulator (see the patent of the RU Nº 2113249, Cl A 61 N 1/36, 1998), containing the first and the second sawtooth, a square-wave generator, a power influence control unit, a trapezoidal generator, a power amplifier, an output unit, the unit for displaying the output signal, active and passive electrodes, where the first adjusting input of the electrostimulator is connected to the adjusting input of the square-wave generator that has a control input connected to the first control input of the electrostimulator, the signal input of the square-wave generator is connected to the signal output of the first sawtooth generator, and the output is connected to clock inputs of the energy influence control unit and of the trapezoidal generator, the second control input of the electrostimulator is connected to the control input of the power influence control unit and with the first control input of a trapezoidal generator, the third control input of the electrostimulator is connected to the second control input of the trapezoidal generator, the second adjusting input of the electrostimulator is connected to the adjusting input of the power influence control unit, that has a signal input connected to the signal output of a trapezoidal generator, and the signal output of the power influence control unit is connected to the signal input of the power amplifier having first and second signal outputs connected to the first and the second signal inputs of the output unit accordingly, and the third signal output is connected to the input of the output signal display unit, the third the fourth adjusting inputs of the electrostimulator are connected to the first and the second adjusting inputs of the output unit accordingly, the fourth control input of the electrostimulator is connected to the control input of the output unit that has the third signal input connected to the signal output of the second sawtooth generator, while the first and the second signal outputs of the output unit are connected to active and passive electrodes accordingly.

The known device has the following drawback: first, it is impossible to adjust the parameters of stimulating pulses depending on the response of skin, and second, it is impossible to control the energy of stimulating pulses taking into consideration the processes of adaptation of a human organism to the influence of stimulating pulses.

The known device neither has the means for realizing the accommodation rule, nor the means for selecting the energy and parameters of stimulating pulses that would in the best way meet the requirements of the "force - duration" curve that is individual for each organism and is varying depending on the stage of disease.

The drawback of the known device consists in distinctive features of structural realization of the known device that allow adjusting only the energy of the pulses, but not allow adjusting the shape of the output signal depending on response of the skin.

There is a known electrostimulator (see AC Nº 2068277, Cl. A61N 1/36, 1996), containing a square pulse unit, an output unit, a stimulating signals value assignment unit, a unit for measuring the duration and the rate of change of duration of the first half wave of the forced oscillations, a one-half-period rectifier, a display and control unit, active and passive electrodes, where the output of the square pulse unit is connected to the first signal input of the output unit, having the second signal input connected to the output of the unitetting the parameters of stimulating signals, having the control input connected to the first control output of the display and control unit, having the second control output connected to the control input of the output unit, having the first and the second signal outputs connected accordingly to the active electrode and the first signal input of the one-half period rectifier, to the passive electrode and the second signal input of the one-half-period rectifier, having a signal output connected to the first signal input of the unit for measuring the duration and the rate of change of duration of the first half wave of the forced oscillations, having the second signal input connected to the third signal output of the output unit, and the output is connected to the signal output of the display and control unit.

The drawback of the known device consists in that depending on the skin response to the influence of stimulating pulses the variation of their parameters occurs according to the measured parameters of the first half wave. However for therapy the analysis of changes of parameters of other half waves is also very important.

In the known device the doctor has no possibility to control the energy of stimulating pulses by shaping the output signal thus having no possibility to reach the best therapeutic effect.

The features of the prototype coinciding with the features of the claimed engineering solution are the output unit, the display and control unit, the active and passive electrodes.

The reasons preventing the known device from achieving the required technical result consist in the features of structural realization of the known device that neither allows to undergo treatment taking into account the analysis of changing parameters of stimulating signals, nor to adjust the shape of stimulating pulses for achieving the best therapeutic effect and inducing anesthesia.

The device that has the technical features most close to the claimed adaptive electrostimulator, is the adaptive electrostimulator (see the Patent RU N 2155614, Cl. A61N 1/36, 2000), containing active and passive electrodes, a unit for feedback signals analysis and an output unit connected to the active electrode,

The known device also contains a square pulse unit, a unit for building the pulse packets, a control unit, a power influence control unit, a unit for storing an individual norm, a unit for recording the parameters of probing signal connected to the first control input of the adaptive electrostimulator.

Between the drawbacks of the known device are a large number of control and adjusting inputs necessary for making initial setting of the device, adjusting and controlling the output signal during the process of therapy. This reduces the operational ability of the device, makes it inconvenient for treatment and the process of treatment itself becomes inefficient since the doctor has to waste time for adjusting the device, recollecting the purpose of buttons. As a whole this reduces the efficiency of therapy.

Another drawback is the absence of possibility of software adjusting the device using a personal computer.

### Disclosure of the invention

The offered invention is targeted on the following technical problems:
- improving the therapeutic effect from using the electrostimulator;
- extending the functionalities of an electrostimulator due to making initial adjustments of the device using a processor unit connected to a computer;
- efficient individual selection by the doctor of stimulating pulses parameters at using a simple and user-friendly interface, that, as a whole, provides improves the treatment effect and optimizes the procedure of treatment.

The technical result in the offered invention is obtained by making an adaptive electrostimulator having an active and a passive electrodes, a unit for analyzing the skin feedback signals and an output unit connected to the active electrode having according to the invention, an additional built-in processor unit, a power supply unit, a power supply control unit, a control keys unit, an indication lights unit, an indication sound unit, an output signal indication unit, a computer interface unit, where the power supply unit is connected to the processor unit, with power supply control unit, with control keys unit, with computer interface unit and with output unit, and the processor unit is connected to power supply control unit, keys control unit, indication lights unit, indication sound unit, unit for analyzing feedback signals of the output unit and the output signal indication unit and a feedback with computer interface unit, where the unit for analyzing the feedback signal is connected to the active electrode and to the output unit, that is connected to the passive electrode.

The invention also is characterized by the presence of an additional built-in unit for monitoring the parameters of the output signal that is connected to the output unit and is equipped with outputs for connecting to a computer.

Thus the unit for monitoring the parameters of the output signal contains the connectors designed for connecting with an output unit and connected to an amplifier that is connected to an alphanumeric converter, to a skin impedance simulation unit and to a shaper that is connected to the processor having a feedback with the computer interface unit and is connected through the alphanumeric converter to a working storage unit having an output connected to the processor, while the processor is connected to the skin impedance simulation unit.

This allows to assess the status of skin or of the area of influence, thus to use it for setting the diagnostic system according to a special algorithm.

Providing the electrostimulator with an additional speech information unit connected to the processor unit using a feedback allows making it available to those having weak eyesight or blind, and to disabled persons who cannot move.

The unit of speech information may be implemented as an additional processor unit connected to a low-pass filter that is connected through a low-frequency amplifier with a dynamic head and through the feedback to read-only memory devices.

The invention also is characterized by the use of a transmitting apparatus that is connected to the processor unit, having an aerial for communication with a remote indicator.

This allows defining the operability of the adaptive electrostimulator by connecting a remote indicator.

Implementing the electrostimulator having microprocessor devices and using an electrostimulator-to-computer interface for bringing a therapy program into the processor, for making initial settings allow taking into account the variety of specific features of patients who require individual selection of stimulating pulses parameters.

Individual selection of stimulating pulses parameters may be done empirically. The doctor should be equipped with a user-friendly interface for a duly and correct choice of stimulating pulses parameters, allowing to change their repetition rate and amplitude, to set an energy level of stimulating pulses.

The electrotherapy goes together with simultaneous disease diagnostics based on the analysis of on change of the stimulating pulses shape in response of an organism to stimulating influences. It is the measurement of duration of the first two half-cycles, the assessment of dynamics of the first, second and third half-cycles, the analysis of asymmetry of pulse parameters in symmetric zones of a body as well as the measurement of the speed of response in the initial phase of influence.

It is obvious that analyzing such a variety of factors with the purpose of disease diagnostics is possible only with using microprocessors and computer facilities. Thus it is appropriate to use a device equipped with digital means for processing the results.

The adaptive electrostimulator allows automatically finding those frequencies rendering the best therapeutic when treating a patient and provides the medical stuff with a user-friendly interface to simplify the treatment process. This is reached by implementing a microprocessor in the proposed embodiment, by using a personal computer interface that in turn allows to vary the parameters of electrostimulator output signals in a wide range and to choose them correctly.

The patent researches have shown that there is no engineering solutions having the specified totality of essential attributes in similar adaptive electrostimulators, i.e. the proposed solution meets the criterion of "novelty".

The analysis of the known analogues and the prototype has not shown the existence of totality of the essential attributes stated in the claim; it means that for specialists in the field of electrotherapy is obviously does not follow from the state of the art thus it meets the criterion of "inventive step".

We consider the information stated in the application enough for practical embodiment of the invention.

### Brief description of figures

The essence of the present invention is illustrated by the following design descriptions and figures where:

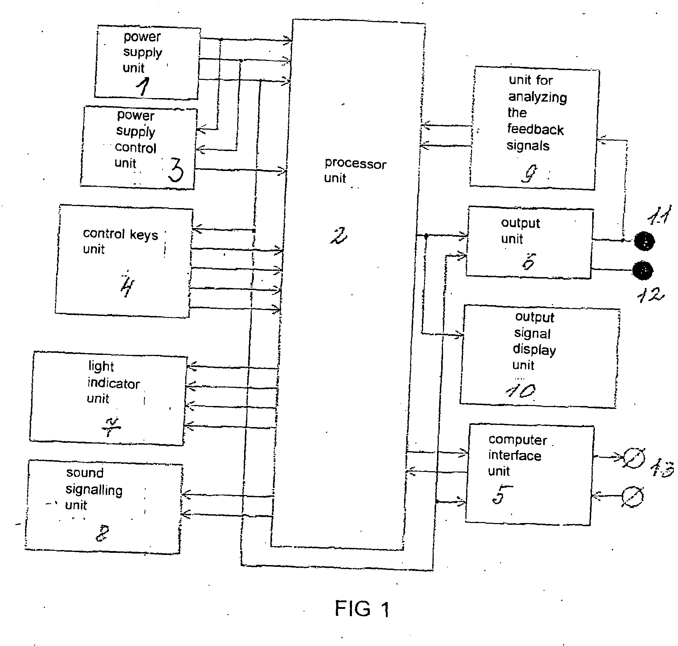

Fig.1 is the block diagram of the adaptive electrostimulator is resulted;

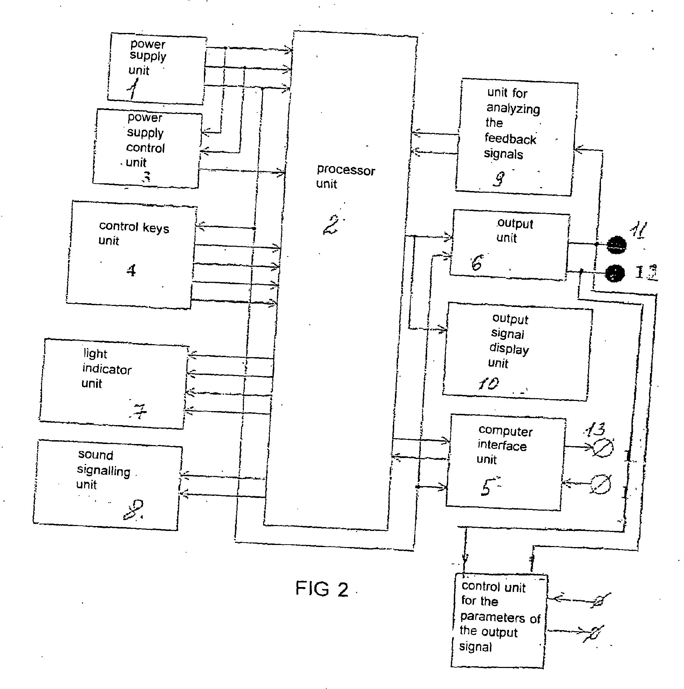

Fig.2 is the block diagram of the adaptive electrostimulator equipped with an output signal parameters control unit;

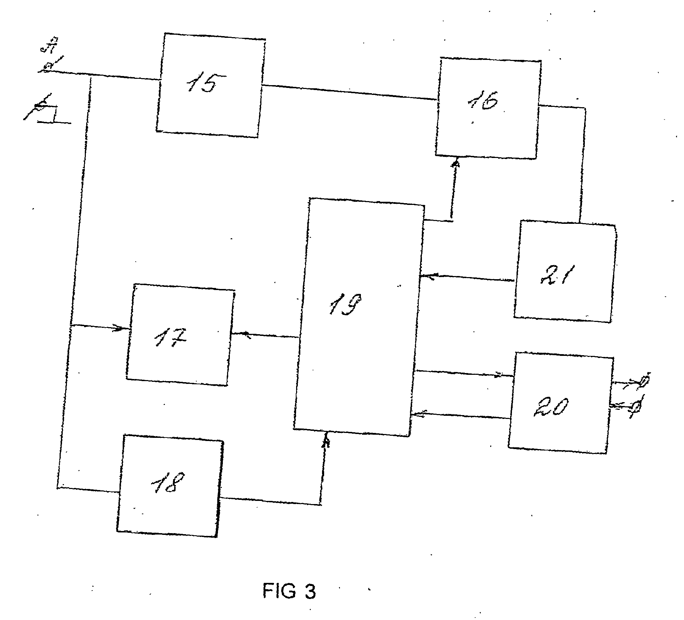

Fig.3 is the block diagram of the output signal parameters control unit;

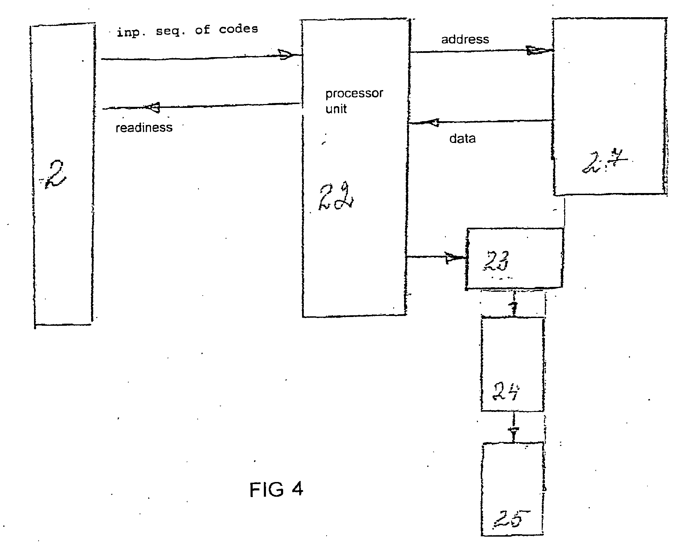

Fig.4 is the block diagram of the speech indication unit;

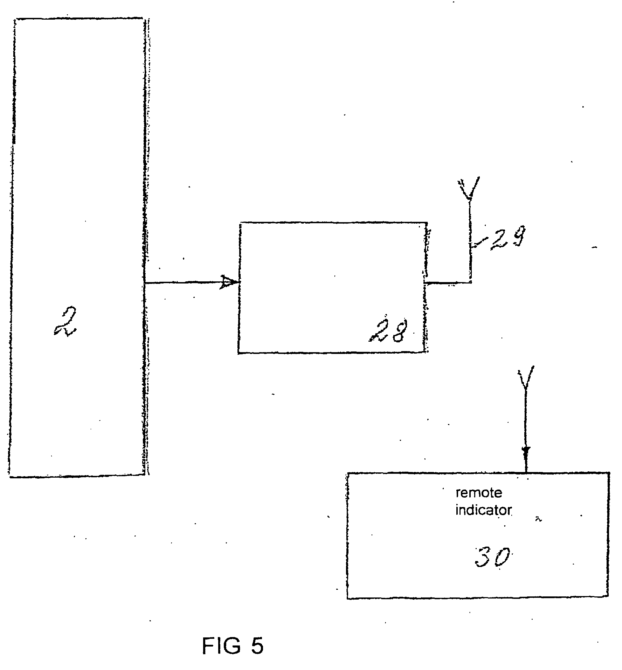

Fig. 5 is the diagram for connecting the remote indication unit.

### The best embodiment

The adaptive electrostimulator containing a power supply unit 1 connected to the processor unit 2, to the power supply control unit 3, with the control keys unit 4, with the computer interface unit 5 and the output unit 6.

The processor unit 2 is connected to the power supply control unit 3, the control keys unit 4, the unit of light indicators 7, the unit of sound signaling 8, the unit for analyzing the feedback signals 9, with the output unit 6 and the output signal display unit 10. The processor unit 2 is connected using feedback to the computer interface unit 5.

The unit for analyzing the feedback signals 9 is connected to the active electrode 11 and to the output unit 6 that is connected to the passive electrode 12.

The adaptive electrostimulator may be additionally equipped with the output signal parameters control unit connected to the output unit 6 and having the outputs 13 for connection to the computer.

The output signal parameters control unit contains the connectors 14 designed for connecting to the output unit 6 and connected to the amplifier 15 that is connected to the alphanumeric converter 16, the skin impedance simulation unit 17 and the shaper 18.

The shaper 18 is connected to the processor of the unit 19 having feedback with the computer interface unit 20 and through the alphanumeric converter 16 to the working memory 21 that has an output connected to the processor 19.

The processor 19 is connected to the skin impedance simulation unit 17.

In case of use of this electrostimulator for people with weak sight or blind as well as for disabled persons who cannot move, it has an additional built-in unit of speech information connected by feedback to the processor unit 2.

The unit of speech information may be implemented as an additional processor unit 22 connected to the low-pass filter 23 that is connected through the low-frequency amplifier 24 to the dynamic head 25 and through feedback with the read-only memory 26 and with the read-only memory 27.

To make the adaptive electrostimulator available for checks of its operability, it is equipped with the transmitting device 28 connected to the processor unit 2 and the aerial 29 designed for communication with the remote indicator 30.

The remote indicator may be of any known design, it is an independent device that is not included in the adaptive electrostimulator.

The adaptive electrostimulator operates as follows:

In physiotherapy there is a way of treating functional disorders having the somatic etiology caused by neuralgic diseases by application of therapeutic influence with electric signals.

There is a known curve of neurology (see M.Brezhe «Electric activity of nervous system», Mir Publishers, 1979. p. 30), showing the threshold of "sensitivity" of nerve fibres depending on the energy and duration of influence. In case of pathology this curve is offset.

Researches have shown that when influencing a human body with stimulating pulses having the parameters that are lower than the threshold level, there will be no response (the action potential is absent) even if the energy of stimulus is high.

When influencing the skin with stimulating pulses exceeding threshold values the response will be similar irrespective of their energy. It is caused by the internal metabolism of cells.

Thus the influence using a pulse having significant energy can cause painful sensations and unwanted responses of the organism. There is a known effect of accommodation named the effect of E. Du Bois-Reymond that shows that the response of stimulated tissues depends on the energy of influence and on the rate of its change as well.

Thus the duly and correct choice of shape and duration of stimulating pulses should meet the specific features of an organism.

The most efficient stimulating influence is the stimulating influence using extremely short pulses having the necessary energy to call the response of a cell. In this case the effect of therapeutic influence will be ensured in the best way.

The criteria for choosing the pulse parameters can be base only on physiological information obtained after following up the patient.

For preparing the electrostimulator for operation there is a software installed on a computer.

The electrostimulator is connected to a serial port of a computer using the computer interface unit 5.

After switching on the control keys unit 4 the electrostimulator and the processor unit 2 are powered up.

The program is recorded from the computer in the memory of the processor unit 2; this program further controls the operation of the adaptive electrostimulator.

The event is being analyzed in the processor unit 2: if the operation began or if there were no powering up after switching the control keys unit 4 on.

If the powering up is to be done for the first time after replacing the power supply the unit of sound signaling 8 will give a sound signal by forming corresponding signals in the processor unit 2. The signal tone corresponds to the sound of the first powering up.

The signal in the unit of sound signaling 8 is formed in the processor of the processor unit 2 for each message separately.

The second signal from the processor unit 2 is the signal of resolution. As the signal of resolution is a binary "one" the first element AND NOT acts as a buffer element, and the second element AND NOT is the factor of voltage.

Then in the processor of the processor unit 2 sets the values of the initial parameters of the processor itself.

If the powering up in the control keys unit 4 is not the first event in time after replacing the power supply the unit of sound signaling 8 gives a sound having the tone of repeated powering up the electrostimulator and the indication of previously chosen operating modes occurs.

During the first switching-on of the control keys unit 4 the operation in the initialization mode is determined. The actual mode is displayed by switching-on a light-emitting diode of the unit of light display 7, where if a LED of the unit of light display 7 is switched on, switching-on one of keys of the control keys unit 4, will switch on the initialization mode and pressing another key of the control keys unit 4 at this time will switch the initialization mode off.

The display of modes by light-emitting diodes of the unit of light display 7 occurs some time after pressing the control keys unit 4 for its deenergizing; then the chosen light-emitting diode goes off and the adaptive electrostimulator automatically switches to the mode of setting the energy of output signal.

After that the processor of the processor unit 2 carries out the control of the power supply.

The battery power is being monitored continuously during the operation of the electrostimulator.

If the voltage goes down to the level of 7.0 to 7.5 V the unit of sound signaling 8 will give a sound signal having a tone set by the processor of the processor unit 2. It means that it is necessary to replace the battery.

Monitoring the battery power is carried out by the power supply control unit 3. The power supply control unit 3 divides the voltage for comparing it with the reference voltage that is fed to the power supply control unit 3.

The processor unit 2 is soft set to a certain level. If the voltage goes down to the level of 7.0 to 7.5 V the power supply control unit 3 feeds the corresponding level of voltage to the processor unit 2, the processor of the processor unit 2 recognizes this level and send a corresponding signal to the unit of sound signaling 8.

If the power supply control unit 3 defines the voltage level to be sufficient the processor of the processor unit 2 checks the communication with the personal computer.

If the communication with the computer is established, the operation mode parameters of the electrostimulator are entered.

If the communication with the computer is not established, polling the control keys unit 4 is carried out.

The control keys unit may have three keys (not shown in the figure):
the key 31 for choosing the settings mode;
the key 32 increasing the parameter of the chosen setting mode;
the key 33 reducing the parameter of the chosen setting mode.

The mode chosen using the key 31 is displayed by the light-emitting diodes of the unit of light display 7.

Choosing between the setting modes is made by successive pressing the key 31 or the key 32 (rising the parameter), or the key 33 (lowering the parameter).

The second pressing of the key 31 chooses the mode of setting the output signal frequency. This mode is displayed by the light-emitting diode of the unit of light display 7.

If the key 31 is pressed when the light-emitting diode is on, the output signal frequency rises.

If the key 32 is pressed when the light-emitting diode is on, the output signal frequency goes down. The output signal frequency is the pulse repetition rate.

When pressing the keys 30, 31, 32 of the keys control unit 4 for the third time (consecutively) the sweep frequency mode of the output signals will be chosen.

Pressing the keys 30, 31 of the keys control unit 4 for the fourth time chooses the amplitude modulation mode of output signals. This mode is displayed by lighting on of one of light-emitting diodes of the unit of light display 7. Pushing the key 33 of the control keys unit 4 switches off the amplitude modulation mode of the output signal.

The stimulating pulses fed to the patient's skin by connecting the active 11 and the passive 12 electrodes to the skin, are shaped in the output unit 6.

If none of the keys 30, 31, 33 control keys unit 4 is pressed, the timer of deenergizing the electrostimulator starts.

The electrostimulator deenergizes upon meeting one of the two conditions:
- The timer of deenergizing has stopped the operation of the device;
- A certain combination of keys 30 to 33 of the control keys unit 4 is entered.

Any change of status of keys the 30 to 33 restarts the timer (not shown in the figure) of deenergizing the electrostimulator.

If there is no deenergizing upon expiration of the pause, the parameters of stimulating influence are to be entered in the processor unit 2 manually using the control keys unit 4.

The procedure of input has been described above. The doctor has the possibility to choose the appropriate influence by stimulating pulses on the chosen area of the patient's skin at doctor's discretion.

Then the processor of the processor unit 2 calculates the parameters of stimulating influence pulses, i.e. defines the level of energy of influence by stimulating pulse, the frequency of pulses, the parameters of amplitude modulation.

According to the made calculations the signals having the shape of pulses to be sent from the processor unit 2 to output unit 6 are formed.

The pulse duration Δt in the processor unit 2 defines the energy of stimulating pulses.

The output unit 6 provides for protection of the processor of the processor unit 2 from disruption.

The pulses having negative polarity from the output unit 6 are fed to the processor unit and open it.

When the electrodes 8 and 10 are not connected there will be free oscillations (absence of load) in the output unit 9 and when applying the electrodes 11 and 12 to the patient's skin, the oscillations will be forced and their waveform will depend on the status of tissues and organs of the patient (forced oscillations).

Intensity of luminescence of the output signal display unit 10 depends on the current flowing through the processor unit 2 to the output unit 6.

Using the unit for analyzing the feedback signals 9 the signals allowing to measure the duration of half waves and the number of crossings in stimulating pulses are fed to the processor unit 2.

The processes occurring in the processor unit are similar to the known, and are not a subject of the invention.

The processor of the processor unit 2 checks the presence of load on the electrodes 11 and 12.

If electrodes are not in contact with the patient's skin, the processor of the processor unit 2 monitors the absence of pulses on the inputs of the processor unit during a preset time.

After this time is over it takes a decision that the time of therapy has expired and the electrostimulator is switched off

If during the preset time the electrodes 11 and 12 begin to contact of with the patient's body then the next part of the working program begins to work, and further it is possible to either switch off the electrostimulator or to enter new parameters of stimulating pulses as it has been described above.

If the electrostimulator is applied to the patient and it is the first application then the diagnostics threshold values are defined, i.e. distinction between the first N pulses and the last pulse are preset for the number of crossings of the X axis by forced oscillations pulses, as well as the distinction between the first N pulses and the last pulse for each i-th half wave of the stimulating pulse.

Than the process of stimulation begins as well as the process of monitoring the attainment of diagnostics threshold during the electrostimulation.

If it is not the first application then the device switches over to the next program and monitors the achievement of the preset thresholds.

If the distinction between the first N pulses and the last pulse for the number of crossings of the X axis by forced oscillations pulses, as well as the distinction between the first N pulses and the last pulse for each i-th half wave of the stimulating pulse are within the preset limits (the thresholds are achieved), the unit of sound signaling 8 gives discontinuous audio signal set by the processor unit 2, and the light-emitting diodes in the unit of light indicators 7 begin to glow in series.

Then the device switches over to the next program and it is possible either to switch off the electrostimulator, or to enter new parameters.

If the distinction between the first N pulses and the last pulse for the number of crossings of the X axis by forced oscillations pulses, as well as the distinction between the first N pulses and the last pulse for each i-th half wave of the stimulating pulse are not within the preset limits (the thresholds are not achieved), the level of achievement of thresholds is displayed.

The display is as follows.

If the difference in settings is within 25% one light-emitting diode of the unit of light indicators 7 lights up.

If the difference in settings is within 50% two light-emitting diodes of the unit of light indicators 7 light up.

If the difference in settings is within 75% all the three light-emitting diodes of the unit of light indicators 7 light up.

Then the necessity of correction of thresholds is being checked.

Decision-making is done on the time factors basis (over certain time interval).

If there is no need for correcting the thresholds then it is possible either to switch off the electrostimulator, or to enter new parameters as it has been described above.

If the processor 17 of processor unit 1 has defined that it is necessary to correct the thresholds then they are being corrected.

The values of distinctions between the first N pulses and the last pulse for the number of crossings of the X axis by forced oscillations pulses, as well as the distinction between the first N pulses and the last pulse for each i-th half wave of the stimulating pulse are being re-calculated according to the set above rule and the process of continues till the achievement of results.

The adaptive electrostimulator may be equipped with an output signal monitoring unit, connected to unit of feedback signals analysis 9 and to the output unit 6 and having outputs 34 for connecting to a computer.

This unit is required for testing the state of patient's skin or the area of influence, for further adjusting the operation of the device.

The output signal monitoring unit operates as follows:

First a signal is generated using the shaper 18, where this signal has a leading edge used by the processor of the unit 19 for generating a sampling frequency for the alphanumeric converter 16.

The processor 19 allows to make the preset number of execute the set number of analog-to-digital signal conversions transformations and to register it in the write down in the working memory 21.

These results are fed through the computer interface unit of the parameters control unit to the computer for soft processing the start and the end of analog signal conversion.

The processed information allows to adjust the output unit 6, and to monitor the operation of the electrostimulator under a load and without it.

When monitoring the operation of the electrostimulator under a load, the skin impedance simulation unit is connected. According to the indications of this unit the device undergoes full adjustment; it chooses itself the required modes, the time and the doze of influence to the skin.

If the device is equipped with a unit of speech information, the latter operates as follows:

In the proposed embodiment the processor unit 2 controls its operation.

When analyzing the readiness to receive orders, the processor 2 forms the code of a word or of a phrase that is required to be pronounced at the moment.

The processor unit 2, having received through the channel 34 the code of a word or phrase, forms the initial address of this word for the read-only memory of speech fragments 26.

Then the previously registered code values corresponding to the instant voltage values of a word or a phrase are red out from the unit 26 in series.

Further the processor unit 22 forms a pulse-width signal having a constant clock frequency (pulse-width modulation). The processor unit 22 is connected to the low-frequency amplifier through the low-pass filter 23 for suppressing the components in the pulse-width modulation spectrum multiple or equal to the clock frequency, and the low-frequency amplifier amplifies the generated sound signal to the values required for operation of the dynamic head 25.

After the word or a phrase have resounded, the device is ready for receiving the following code of a word or a phrase.

When changing the operating modes, and when changing the parameters of stimulating pulses in the diagnostics mode the processor unit 2 forms a consecutive code of information for the remote indicator 30. These consecutive codes are being fed to the transmitting device 28. The modulated radio-frequency oscillations are radiated by the aerial of the transmitting device 29; further those oscillations are received by the remote indicator 30 that defines the operability of the device.

### Industrial applicability

In the known device the percutaneous influence by stimulating pulses on a skin area is realized in such a manner that the physician selects initial parameters of amplitude and frequency by manipulating a large number of control keys, while the parameters of analysis algorithms of probing signals are constant.

In the offered device the physician sets the parameters of influence using a small number of keys, while the parameters of analysis algorithms of probing signals may be chosen using special software way from the keyboard of a personal computer at the stage of presetting the device.

The adaptive electrostimulator may be realized using the processors available in the international market.

## Claims

1. An adaptive electrostimulator containing active (11) and passive (12) electrodes, a unit for analyzing the feedback signals (9) and an output unit (6) connected to the active electrode (11), whereas it is additionally equipped with a built-in processor unit (2), a power supply unit (1), a power supply control unit (3), a control keys unit (4), a unit of light indicators (7), a unit of sound signaling (8), an output signal display unit (10), a computer interface unit (5), where the power supply unit (1) is connected to the processor unit (2), to the power supply control unit (3), to the control keys unit (4), to the computer interface unit (5) and the output unit (6), while the processor unit (2) is connected to the power supply control unit (3), to the control keys unit (4), to the unit of light indicators (7), to the unit of sound signaling (8), to the unit for analyzing the feedback signals (9) having the output unit (6) and the output signal display unit (10) and the feedback with the computer interface unit (5), and the unit for analyzing the feedback signals (9) is connected to the active (11) electrode and to the output unit (6) that is connected to the passive electrode (12).

2. An adaptive electro stimulator as claimed in Claim 1 whereas it is additionally equipped with an output signal parameters control unit connected to the output unit (6) and having the outputs (13) for communication with computer.

3. An adaptive electrostimulator as claimed in Claim 2 whereas the output signal parameters control unit contains the connectors (14) for connection with the output unit (6) and connected to the amplifier (15), that is connected to the alphanumeric converter (16), the skin impedance simulation unit (17) and the shaper (18) that is connected to the processor (19) having a feedback with the computer interface unit (20) and through the alphanumeric converter (16) with working memory (21) having an output connected to the processor (19), where the processor is connected to the skin impedance simulation unit (17).

4. An adaptive electrostimulator as claimed in Claim 1 whereas it is additionally equipped with a unit of speech information connected by feedback to the processor unit (2).

5. An adaptive electrostimulator as claimed in Claim 4 whereas the unit of speech information is made as an additional processor unit (22) connected to the low-pass filter (23) that is connected through the low-frequency amplifier (24) to the dynamic head (25) and by feedback to the read-only memory (26) and to the read-only memory (27).

6. An adaptive electrostimulator as claimed in Claim 1 whereas it is equipped with a transmitting device (28), connected to the processor unit (2), having an aerial (29) for communication with the remote indicator (30).
